(19) Europäisches Patentamt
European Patent Office
Office européen des brevets

(11) Numéro de publication: **0 001 940** A1

# (12) DEMANDE DE BREVET EUROPEEN

(21) Numéro de dépôt: **78400137.2**

(22) Date de dépôt: **18.10.78**

(51) Int. Cl.²: **C 07 D 471/22**
//(C07D471/22, 221/00, 221/00, 221/00, 209/00)

(30) Priorité: **02.11.77 FR 7732855**

(43) Date de publication de la demande: **16.05.79 Bulletin 79/10**

(84) Etats contractants désignés: **BE CH DE FR GB LU NL SE**

(71) Demandeur: **SYNTHELABO**
**1, avenue de Villars**
**F-75341 Paris Cedex 07(FR)**

(72) Inventeur: **Koletar Gabor, Istvan**
**212 Dogwood Lane**
**Berkeley Heights New Jersey 07922(US)**

(72) Inventeur: **Najer, Henry**
**2, avenue Emile Acollas**
**F-75007 Paris(FR)**

(72) Inventeur: **Lardenois, Patrick André Louis**
**18, rue Varengues**
**F-92340 Bourg La Reine(FR)**

(72) Inventeur: **Lefevre, Jean Pierre**
**5, rue de Rocroy**
**F-75010 Paris(FR)**

(74) Mandataire: **Thouret-Lemaitre, Elisabeth**
**Service Brevets - SYNTHELABO 58, rue de la Glacière**
**F-75621 Paris Cedex 13(FR)**

(54) **Dérivés de l'éburnaménine, leur préparation et leur application en thérapeutique.**

(57) Dérivés de l'éburnaménine de formule:

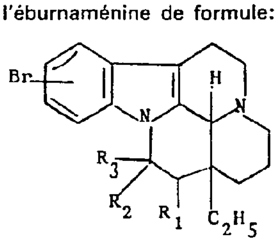

dans laquelle
$R_1$ = H et $R_2$ = OH ou $R_1$ + $R_2$ = liaison supplémentaire; $R_3$ = $COOR_4$ (avec $R_4$ = alkyle de 2 à 6 atomes de carbone ou un cycloalkyle) ou $CONR_5R_6$.

Soit $R_1$ = $R_2$ = H et $R_3$ = $COOR_7$ (avec $R_7$ = alkyle de 1 à 6 atomes de carbone ou un cycloalkyle) ou $CONR_5R_6$. $R_5$ et $R_6$ = H, alkyle, cycloalkyle ou forment ensemble avec l'atome d'azote un radical hétérocyclique. La première étape de leur préparation est une bromuration. Les composés possèdent une activité anti-anoxique et psychotrope.

EP 0 001 940 A1

Dérivés de l'éburnaménine leur préparation et leur application en thérapeutique.

La présente invention a pour objet de nouveaux dérivés de l'éburnaménine, leur préparation et leur application en thérapeutique.

Les composés de l'invention répondent à la formule (I)

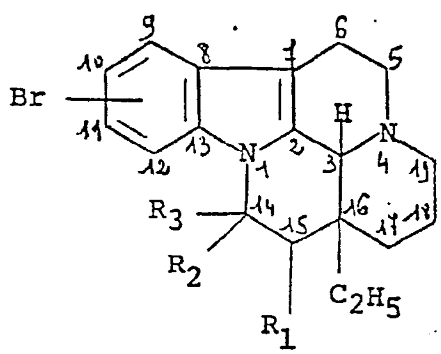

(I)

dans laquelle

- lorsque $R_1$ représente H et $R_2$ représente OH ou lorsque $R_1$ et $R_2$ représentent ensemble une double liaison, $R_3$ représente un groupe $COOR_4$ ou $CONR_5R_6$ dans lesquels $R_4$ est un alkyle de 2 à 6 atomes de carbone ou un cycloalkyle de 3 à 6 atomes de carbone et $R_5$ et $R_6$ sont indépendamment l'un de l'autre un atome d'hydrogène, un alkyle de 1 à 6 atomes de carbone ou un cycloalkyle de 3 à 6 atomes de carbone ou $NR_5R_6$ forment ensemble un radical hétérocyclique pouvant ou non comporter un autre hétéro-atome,

- lorsque $R_1$ et $R_2$ représentent chacun un atome d'hydrogène, $R_3$ représente un groupe $COOR_7$ ou $CONR_5R_6$ dans lesquels $R_7$ est un alkyle de 1 à 6 atomes de carbone ou un cycloalkyle de 3 à 6 atomes de carbone et $R_5$ et $R_6$ sont indépendamment l'un de l'autre un atome d'hydrogène, un alkyle de 1 à 6 atomes de carbone ou un cycloalkyle de 3 à 6 atomes de carbone ou $NR_5R_6$ forment ensemble un radical hétérocyclique pouvant ou non comporter un autre hétéro-atome.

Les composés de formule (I) peuvent exister sous la forme d'épimères en $C_{14}$, mais on utilise de préférence l'épimère dans lequel $R_3$ est en $\alpha$.

Les composés de formule (I) peuvent également exister sous la forme d'isomères optiques et de racémiques ($C_3$, $C_{16}$).

Selon l'invention on prépare les composés de formule (I) de la manière suivante :
on brome directement un ester vincaminique ($R_1$ = H, $R_2$=OH, $R_3$ = $COOR_4$), ou désoxyvincaminique ($R_1$ et $R_2$ = H, $R_3$ = $COOR_7$), puis si on le désire, on hydrolyse cet ester en acide bromé que l'on amidifie de manière classique en amide bromé ou si on le désire on déshydrate l'ester vincaminique bromé en ester apovincaminique bromé ($R_1$ et $R_2$ = double liaison).

La bromuration directe de l'ester vincaminique ou désoxyvincaminique est effectuée en présence d'un acide de Lewis dans un solvant chloré, tel que le chloroforme, le tétrachlorure de carbone, ou nitré tel que le nitrobenzène ou le nitrométhane, à une température de 0 à 30°C.
On obtient un mélange des 3 isomères bromés en 9, 10 et 11 que l'on sépare par chromatographie sur colonne.

L'amidification de l'acide vincaminique ou désoxyvincaminique bromé est effectuée de manière classique (obtention du chlorure d'acide par réaction avec SO $Cl_2$ puis amidification à l'aide de l'amine HN $R_5$ $R_6$).

La déshydratation du composé vincaminique bromé (ester ou acide) est effectuée de manière classique à l'aide d'acide formique.

Les composés de l'invention peuvent également être préparés par synthèse totale à partir des tryptamines bromées par une des méthodes de synthèse totale connue.

Les exemples suivants illustrent la présente invention. Les analyses et spectres IR et RMN ont confirmé la structure des composés.

Exemple 1

Bromo-9, bromo-10 et bromo-11 désoxyvincamines.

On met 100 g (0,295 mole) de désoxyvincamine (mélange des deux épimères en $C_{14}$) en solution dans 800 ml de nitrométhane. On refroidit à 0° et on ajoute une solution filtrée de 96 g de Fe $Cl_3$, 6 $H_2O$ dans 800 ml de nitrométhane. On verse ensuite 21 ml de brome goutte à goutte, l'addition se faisant en 4 heures. A la fin de l'addition on verse 800 ml d'eau et on agite 15 mn. La solution organique est décantée et lavée 2 fois avec de l'eau. On la sèche sur $Na_2$ $SO_4$ et l'évapore au bain marie sous vide. On reprend le résidu par 200 ml d'eau et 300 ml de chlorure de méthylène. On neutralise avec $NH_4OH$ 2N. On décante puis évapore la solution organique.
On obtient un résidu huileux composé des isomères bromés en 9, 10 et 11 des épimères en $C_{14}$.

On hydrolyse le mélange des différents composés à l'aide de KOH puis on réestérifie en milieu acide selon la méthode de Fischer pour isoler les dérivés bromés du seul épimère dans lequel $R_3$ est en $\alpha$. Après l'estérification par du méthanol en milieu acide sulfurique, on obtient un précipité (fraction A) que l'on filtre et un filtrat (fraction B).

La fraction A, après neutralisation par l'ammoniaque, purification et recristallisation fournit la bromo-10 désoxyvincamine.

$$F = 190°C \qquad \alpha_D^{20} \ (1\%, CHCl_3) = + 118°$$

Après neutralisation par l'ammoniaque, la fraction B contenant un mélange des 3 isomères bromés en 9, 10 et 11 est chromatographiée sur silice (éluant : acétate d'éthyle/méthanol 80/20).

On sépare les bromo-9 et bromo-11 désoxyvincamines.

La bromo-9 désoxyvincamine est recristallisée dans de l'éthanol.

$$F = 168\text{-}170°C \qquad \alpha_D^{25} \ (1\%, CHCl_3) = + 132,6°$$

<u>Exemple 2</u>

Bromo-10 désoxyvincaminamide.

Dans un ballon avec garde à chlorure de calcium on place 1,6 g (0,004 mole) d'acide bromo-10 désoxyvincaminique obtenu par hydrolyse basique de la bromo-10 désoxyvincamine purifiée et 60ml de benzène anhydre. On ajoute 0,4 ml de pyridine puis 0,4 ml de $SO\ Cl_2$ (0,004 mole). On agite le

mélange réactionel une nuit à la température ordinaire. On obtient le chlorure d'acide.

On fait ensuite barboter $NH_3$ pendant 3 heures en chauffant à 80° (température du bain d'huile). Après refroidissement on ajoute 50 ml d'une solution de $NH_4OH$ 2N. On agite, décante la phase organique, la lave à l'eau et l'évapore au bain marie sous vide.
Le résidu est purifié par passage sur 30 g d'alumine. On élue avec $CH_2Cl_2$.
On obtient après évaporation du chlorure de méthylène un résidu blanc que l'on recristallise dans de l'éther. On obtient ainsi le bromo-10 déxosyvincaminamide.

$$F = 270°C \qquad \alpha_D^{20} (1\%, CHCl_3) = + 91,5°$$

Exemple 3

Bromo-10 et bromo-9 vincaminates de n-butyle.

A une suspension de 32,5 g de $FeCl_3$, 6 $H_2O$, dans 250 ml de nitrométhane, de laquelle on décante une huile, on ajoute lentement 7 ml de brome. La solution ainsi obtenue est alors ajoutée goutte à goutte à une autre solution refroidie à 0° de 39,6 g (100 m moles) de vincaminate de n-butyle (préparé par estérification de l'acide vincaminique par le bromure de n-butyle dans EtOH-HMPT en milieu KOH), dans 250 ml de nitrométhane.
L'introduction terminé, on agite encore 30mn à 0°, puis après avoir laissé remonter à la température ambiante, on ajoute 250 ml d'eau.
On décante la phase organique, que l'on amène à sec, que l'on reprend par $H_2O$, puis que l'on alcalinise par $NH_4OH$.

On obtient ainsi une concrétisation. On essore et isole un mélange des 3 dérivés bromés en 9, 10 et 11. Ce mélange, par recristallisations successives dans de l'éther éthylique avec passage au noir fournit le bromo-10 vincaminate de n-butyle.

F = 160,5°-161,5°C $\alpha_D^{20}$ (1% $CHCl_3$) = + 62°

Les eaux mères de la recristallisation sont chromatographiées sur silice puis éluées par AcOEt à 10% d'acétone. On obtient le bromo-9 vincaminate de n-butyle.

F = 128-130°C $\alpha_D^{20}$ (1% $CHCl_3$) = + 45,1°

Exemple 4

Bromo-9, bromo-10 et bromo-11 apovincaminates de n-butyle.

On agite, pendant 16 heures au reflux, une solution de 10g du mélange de bromo-9, 10 et 11 vincaminates de butyle dans 100 ml d'acide formique à 99%. En fin de reflux, on refroidit, verse le mélange sur 1000 ml d'eau, puis alcalinise par $NH_4OH$.
On extrait la gomme obtenue plusieurs fois par $CHCl_3$. On réunit les phases organiques, qu'on lave à l'eau, sèche sur $SO_4Na_2$, filtre et concentre à sec. On chromatographie sur silice avec comme éluant le mélange acétone-1, AcOEt-1, $CHCl_3$-1, on obtient :

a) un composé qui, recristallisé dans de l'éthanol, est le bromo-9 apovincaminate de n-butyle :

F = 130-130,5°C $\alpha_D^{25}$ (1%,$CHCl_3$) = + 175°8

b) après la transformation en chlorhydrate par HCl dans MeOH et recristallisation dans AcOEt avec passage au noir le chlorhydrate du bromo-10 apovincaminate de n-butyle.

$$F = 226\text{-}228°C \qquad \alpha_D^{20} \text{ (1\%, pyridine)} = +\ 67{,}3°$$

c) après transformation en chlorhydrate par HCl dans MeOH le chlorhydrate du bromo-11 apovincaminate de n-butyle.

$$F = 214\text{-}216°C \qquad \alpha_D^{20} \text{ (1\% pyridine)} = +\ 32{,}4°$$

Dans le tableau I suivant sont rassemblés les composés de l'invention préparés à titre d'exemples.

m.s = méthane sulfonate
c = chlorhydrate

TABLEAU I

| Composé | $R_1$ | $R_2$ | $R_3$ | Br position | F (°C) | α (°) |
|---|---|---|---|---|---|---|
| 1 (ex1) | H | H | $COOCH_3$ | 9 | 168 - 170 | $\alpha_D^{25}$ (1% $CHCl_3$) = +132,6 |
| 2 (ex1) | H | H | $COOCH_3$ | 10 | 190 | $\alpha_D^{25}$ (1% $CHCl_3$) = + 118 |
| 3 | H | H | $COOC_2H_5$ | 9 | 136 - 138 | $\alpha_D^{25}$ (1% $CHCl_3$) = + 136 |
| 4 | H | H | $COOC_2H_5$ | 10 | 137 - 139<br>m.s 190-195 | $\alpha_D^{25}$ (1% $CHCl_3$) = + 123,7<br>$\alpha_D^{25}$ (1% $CHCl_3$) = + 89,1 |
| 5 | H | H | $COOCH(CH_3)_2$ | 10 | 140 - 142 | $\alpha_D^{25}$ (1% $CHCl_3$) = + 125,7 |
| 6 (ex2) | H | H | $CONH_2$ | 10 | 270 | $\alpha_D^{25}$ (1% $CHCl_3$) = + 91,5 |
| 7 | H | H | $CONHCH_3$ | 10 | 183 | $\alpha_D^{25}$ (1% $CHCl_3$) = + 119,2 |

TABLEAU I (suite)

| Composé | $R_1$ | $R_2$ | $R_3$ | Br position | F (°C) | α (°) |
|---|---|---|---|---|---|---|
| 8 | H | H | $CON(C_2H_5)_2$ | 10 | 181 - 183 | $\alpha_D^{25}$ (1% $CHCl_3$) = + 55,9 |
| 9 | H | H | CONH 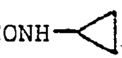 | 10 | 219 - 221 | $\alpha_D^{25}$ (1% $CHCl_3$) = + 145,6 |
| 10 | H | H | CON 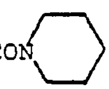 | 10 | 206 - 207 | $\alpha_D^{25}$ (1% $CHCl_3$) = + 67,6 |
| 11 | H | H | CON 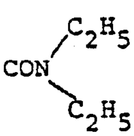 | 11 | 161 - 163 | $\alpha_D^{25}$ (1% $CHCl_3$) = + 6,8 |
| 12 | H | OH | $COOC_4H_{9n}$ | 9 | 128 - 130 | $\alpha_D^{20}$ (1% $CHCl_3$) = + 45,1 |
| 13 | H | OH | $COOC_4H_{9n}$ | 10 | 160,5-161,5 | $\alpha_D^{20}$ (1% $CHCl_3$) = + 62 |
| 14 | H | OH | $COO\ C_2H_5$ | 10 | 222 - 223 | $\alpha_D^{20}$ (1% $CHCl_3$) = + 59,5 |

TABLEAU I (fin)

| Composé | $R_1$ | $R_2$ | $R_3$ | Br position | F (°C) | $\alpha$(°) |
|---|---|---|---|---|---|---|
| 15 (ex3) | double liaison | | $COOC_4H_{9n}$ | 9 | 130 - 130,5 | $\alpha_D^{25}$ (1% $CHCl_3$) = + 175,8 |
| 16 (ex3) | double liaison | | $COOC_4H_{9n}$ | 10 | c=226 - 228 | $\alpha_D^{20}$ (1% pyridine) = + 67,3 |
| 17 (ex3) | double liaison | | $COOC_4H_{9n}$ | 11 | c=214 - 216 | $\alpha_D^{20}$ (1% pyridine) = + 32,4 |
| 18 | double liaison | | $COOC_2H_5$ | 10 | 112 - 113 | $\alpha_D^{20}$ (0,7% $CHCl_3$) = + 80,6 |
| 19 | double liaison | | $COOC_2H_5$ | 11 | c = 216 | $\alpha_D^{20}$ (1% pyridine) = + 43 |

Les composés de l'invention ont fait l'objet d'une étude pharmacologique.

La toxicité a été déterminée chez des souris de souche CD1 par méthode graphique.

La DL 50 des composés de l'invention est de l'ordre de 200 à 500 mg/kg par voie i.p.

Anoxie hypobare

Des souris de souche CD1 sont maintenues dans une atmosphère appauvrie en oxygène, par réalisation d'un vide partiel (190mm de mercure correspondant à 5,25% d'oxygène).

Le temps de survie des animaux est noté. Ce temps est augmenté par les agents capables de favoriser l'oxygénation tissulaire et en particulier cérébrale. Les composés étudiés sont administrés, à plusieurs doses, par voie intrapéritonéale, 10 minutes avant l'essai. Les pourcentages d'augmentation du temps de survie par rapport aux valeurs obtenues chez les animaux témoins sont calculés. La dose active moyenne (DAM), dose qui augmente le temps de survie de 100% est déterminée graphiquement

Les résultats sont indiqués dans le tableau II suivant pour un nombre représentatif de composés.

Action sur la durée du "sommeil" induit par le 4-hydroxy-butyrate de sodium.

Cette action a été déterminée par l'influence des composés sur la durée du "sommeil" induit par le 4-hydroxy-butyrate de sodium (G H B) chez le rat curarisé.

Les animaux utilisés sont des rats mâles de souche Charles River de 200 ± 20 g. Les animaux curarisés par l'alloférine à raison de 1mg/kg par voie i.p. sont

placés sous respiration artificielle à l'aide d'un masque appliqué sur le museau (fréquence respiratoire : 40/minute : volume respiratoire : 10cm3). L'oesophage est préalablement ligaturé afin d'éviter l'entrée de l'air dans l'estomac.

Des électrodes corticales front-pariétales et occipitales permettent l'enregistrement de l'activité électrocorticographique sur un polygraphe Grass modèle 79 P à la vitesse de 6 mm/sec. La préparation de l'animal est effectuée sous anesthésie locale (xylocaïne à 2%). Les rats sont maintenus tout au long de l'expérience à température constante (37°C). Dix minutes après la fin de la préparation du rat, une dose de 200 mg/kg de 4-hydroxy-butyrate de Na est injectée par voie intraveineuse au niveau de la queue.

Des doses de 10 mg/kg des composés à étudier sont administrées par voie intrapéritonéale 3 minutes après l'administration du 4-hydroxy-butyrate de sodium.

L'évaluation des tracés s'effectue par période de 15 minutes durant 75 minutes après l'injection de "G H B". Durant cette période d'analyse, la durée totale du "sommeil" est déterminée. Une série de 15 témoins permet de préciser la durée du "sommeil G H B".

L'analyse statistique des résultats est réalisée à l'aide du test "U" de Mann-Whitney.

Les résultats sont rapportés dans le tableau III suivant

TABLEAU II

| Composé | Anoxie hypobare DAM - i.p. (mg/kg) |
|---|---|
| 13 | 3 - 4 |
| 2 | 3 |
| 6 | 1 |
| 7 | 2 |
| 14 | 3 |
| 5 | 3 |
| 9 | 0,5 - 1 |
| 4 | 1 |
| 8 | 4 |
| 1 | 8 |
| 3 | 8 |
| 18 | 7 |

TABLEAU III

| Composé | Activité sur sommeil<br>Ecart en % par rapport aux témoins |
|---|---|
| 2 | - 41 |
| 6 | - 51 |
| 7 | - 48 |
| 14 | - 36 |
| 15 | - 19 |
| 5 | - 32 |
| 4 | - 29 |
| 8 | - 36 |
| 18 | - 21 |
| 17 | - 27 |
| 16 | - 20 |
| 19 | - 22 |

L'étude pharmacologique des composés de l'invention montre qu'ils sont actifs dans l'épreuve d'anoxie hypobare chez la souris tout en n'étant que peu toxiques et qu'ils exercent une action significative éveillante dans le test du "sommeil" induit par le 4-hydroxy-butyrate de sodium.

Les composés de l'invention, possédant à la fois une activité antianoxique et une activité psychotrope, peuvent être utilisés en thérapeutique pour le traitement des troubles de la vigilance, en particulier pour lutter contre les troubles du comportement imputables à des dommages vasculaires cérébraux et à la sclérose cérébrale en gériatrie, ainsi que pour le traitement des absences dues à des traumatismes crâniens, et le traitement des états dépressifs.

L'invention comprend par conséquent, toutes compositions pharmaceutiques renfermant les composés et/ou leurs sels comme principes actifs, en association avec tous excipients appropriés à leur administration, en particulier par voie orale ou parentérale.

Les voies d'aminístration peuvent être les voies orale et parentérale.

La posologie quotidienne peut aller de 10 à 100 mg.

Revendications de brevet

1. Dérivés de l'éburnaménine, sous la forme d'épimères en $C_{14}$ et sous la forme de racémiques ou d'énantiomères, répondant à la formule

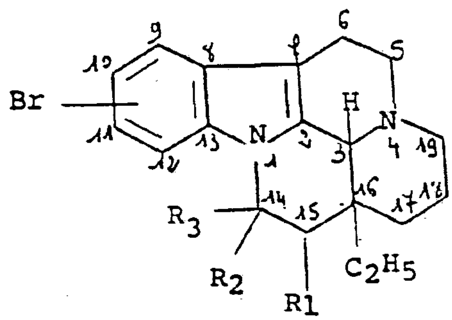

dans laquelle

- lorsque $R_1$ représente H et $R_2$ représente OH ou lorsque $R_1$ et $R_2$ représentent ensemble une double liaison, $R_3$ représente un groupe $COOR_4$ ou $CONR_5R_6$ dans lesquels $R_4$ est un alkyle de 2 à 6 atomes de carbone ou un cycloalkyle de 3 à 6 atomes de carbone et $R_5$ et $R_6$ sont indépendamment l'un de l'autre un atome d'hydrogène, un alkyle de 1 à 6 atomes de carbone ou un cycloalkyle de 3 à 6 atomes de carbone ou $NR_5R_6$ forment ensemble un radical hétérocyclique pouvant ou non comporter un autre hétéro-atome,

- lorsque $R_1$ et $R_2$ représentent chacun un atome d'hydrogène, $R_3$ représente un groupe $COOR_7$ ou $CONR_5R_6$ dans lesquels $R_7$ est un alkyle de 1 à 6 atomes de carbone ou un cycloalkyle de 3 à 6 atomes de carbone et $R_5$ et $R_6$ sont indépendamment l'un de l'autre un atome d'hydrogène, un alkyle de 1 à 6 atomes de carbone ou un cycloalkyle de 3 à 6 atomes de carbone ou $NR_5R_6$ forment ensemble un radical hétérocyclique pouvant ou non comporter un autre hétéro-atome.

2. Composés selon la revendication 1, dans lesquels $R_3$ est en $\alpha$ .

3. Composés selon la revendication 2, dans lesquels l'atome de Br est en position 10.

4. Composés selon la revendication 3, dans lesquels $R_1$ et $R_2$ sont chacun un atome d'hydrogène et $R_3$ représente un groupe $COOR_7$ ou $CONR_5R_6$ dans lesquels $R_7$ est un alkyle de 1 à 6 atomes de carbone ou un cycloalkyle de 3 à 6 atomes de carbone et $R_5$ et $R_6$ sont indépendamment l'un de l'autre un atome d'hydrogène, un alkyle de 1 à 6 atomes de carbone ou un cycloalkyle de 3 à 6 atomes de carbone ou $NR_5R_6$ forment ensemble un radical hétérocyclique pouvant ou non comporter un autre hétéro-atome.

5. Procédé de préparation des composés selon l'une quelconque des revendications 1 à 4, procédé caractérisé en ce qu'on brome directement un ester vincaminique ($R_1$ et $R_2$ = H, $R_3$ = $COOR_4$) ou désoxyvincaminique ( $R_1$ et $R_2$ = H, $R_3$= $COOR_7$), puis si on le désire, on hydrolyse cet ester en acide bromé que l'on amidifie de manière classique en amide bromé ou si on le désire on déshydrate l'ester vincaminique bromé en ester apovincaminique bromé($R_1$ et $R_2$ = double liaison).

6. Médicament caractérisé en ce qu'il contient un composé spécifié dans l'une quelconque des revendications 1 à 4.

Office européen des brevets

# RAPPORT DE RECHERCHE EUROPEENNE

0001940

Numéro de la demande

EP 78 40 0137

| Catégorie | DOCUMENTS CONSIDERES COMME PERTINENTS<br>Citation du document avec indication, en cas de besoin, des parties pertinentes | Revendication concernée | CLASSEMENT DE LA DEMANDE (Int. Cl.²) |
|---|---|---|---|
| | FR - A - 2 254 342 (SANDOZ S.A.)<br>* Revendications *<br>-- | 1,5,6 | C 07 D 471/22/<br>(C 07 D 471/2<br>221/..<br>221/00<br>221/00<br>209/00) |
| A | FR - A - 2 275 211 (SYNTHELABO S.A.)<br>* Revendications *<br>-- | 1 | |
| A | NL - A - 75 08740 (SYNTHELABO S.A.)<br>* Revendications *<br>BE 830 587<br>---- | 1 | DOMAINES TECHNIQUES RECHERCHES (Int. Cl.²)<br>C 07 D 471/22//<br>(C 07 D 471/22<br>221/00<br>221/00<br>221/00<br>209/00) |

CATEGORIE DES DOCUMENTS CITES

- X: particulièrement pertinent
- A: arrière-plan technologique
- O: divulgation non-écrite
- P: document intercalaire
- T: théorie ou principe à la base de l'invention
- E: demande faisant interférence
- D: document cité dans la demande
- L: document cité pour d'autres raisons
- &: membre de la même famille, document correspondant

X Le présent rapport de recherche a été établi pour toutes les revendications

| Lieu de la recherche | Date d'achèvement de la recherche | Examinateur |
|---|---|---|
| La Haye | 29-01-1979 | VAN BIJLEN |